Europäisches Patentamt

(19) European Patent Office

Office européen des brevets

(11) Numéro de publication: **0 283 413**
**B1**

(12) **FASCICULE DE BREVET EUROPEEN**

(45) Date de publication du fascicule du brevet: **06.02.91**

(51) Int. Cl.⁵: **A 61 F 2/24**

(21) Numéro de dépôt: **88420092.4**

(22) Date de dépôt: **17.03.88**

(54) Valve à au moins un volet basculant par rapport à des pivots élastiques.

(30) Priorité: **20.03.87 FR 8704107**

(43) Date de publication de la demande:
**21.09.88 Bulletin 88/38**

(45) Mention de la délivrance du brevet:
**06.02.91 Bulletin 91/06**

(84) Etats contractants désignés:
**AT BE CH DE ES FR GB GR IT LI LU NL SE**

(56) Documents cités:
**US-A-3 448 465**

(73) Titulaire: **Colon, Jean**
**3, rue des Ecoles**
**F-26190 Saint Jean en Royans (FR)**
(73) Titulaire: **Marion, Pierre**
**Tour Carrée Chemin de l'ancienne Eglise**
**Poleymieu**
**F-69250 Neuville sur Saone (FR)**

(72) Inventeur: **Colon, Jean**
**3, rue des Ecoles**
**F-26190 Saint Jean en Royans (FR)**
Inventeur: **Marion, Pierre**
**Tour Carrée Chemin de l'ancienne Eglise**
**Poleymieu**
**F-69250 Neuville sur Saone (FR)**

(74) Mandataire: **Karmin, Roger et al**
**Cabinet MONNIER 150, Cours Lafayette**
**F-69003 Lyon (FR)**

Courier Press, Leamington Spa, England.

## Description

La présente invention est relative à des perfectionnements apportés aux valves à volet basculant utilisées dans des canalisations pour constituer des clapets anti-retour, c'est-à-dire ne permettant le passage d'un fluide que dans un sens. L'invention se réfère plus particulièrement aux prothèses valvulaires cardiaques, car c'est dans ce cas que son application paraît devoir présenter le plus d'intérêt.

On sait que depuis les années 1960, les chirurgiens procèdent au remplacement des orifices du coeur atteints de rétrécissement ou d'insuffisance par des prothèses mécaniques ou biologiques. Les prothèses dites mécaniques, c'est-à-dire à bille, à disque ou à clapet, furent utilisées avant les prothèses dites biologiques (hétéro-greffe prétraitée et supportée par un dispositif rigide).

Les résultats cliniques éloignés basés sur de nombreuses statistiques, ont conduit à deux constats:

1° — Les prothèses mécaniques (celles qui ne perturbent pas l'hémodynamique et qui répondent à de bons tests de fiabilité appréciés sur modèles) ont une durée satisfaisante de fonctionnement supérieure à dix ans et peuvent dépasser vingt ans. Le pourcentage des accidents prématurés est faible (rupture, usure précoce). Par contre, les accidents thrombo-emboliques précoces ou tardifs après la pose d'une prothèse mécanique ne sont pas exceptionnels (évalués entre deux et six pour cent par malade et par an) surtout en position mitrale.

2° — Après la pose d'une prothèse biologique, les accidents thrombo-emboliques sont moins fréquents qu'après la mise en place d'une prothèse mécanique (un à trois pour cent environ, par malade/année). Mais les prothèses biologiques sont moins fiables que les prothèses mécaniques (en moyenne, leur durée de vie se situe en dessous de dix ans). Leur disfonctionnement, très précoce chez l'enfant, est la conséquence de calcifications valvulaires qui provoquent rigidité, sténose et insuffisance.

Parmi les prothèses à clapets, certaines utilisent un dispositif de contention sans charnière, par exemple celle décrite dans le document AU—A—56 266/69. Une telle prothèses a l'inconvénient de nécessiter un dispositif métallique ou plastique fixé sur l'anneau qui est générateur de turbulences ou, éventuellement, d'accrochage des éléments figurés du sang et de la fibrine dans la zone de stase juxta-annulaire de la veine fluide.

D'autres modèles utilsent des volets à charnières. Plusieurs prothèses de ce type sont constituées de deux volets à charnières disposés en ailes de papillon, à ouverture latérale. La charnière est d'un type spécial, telle que celle décrite dans le document FR—A—2 407 709, constituée par un pivot qui, engagé dans une cavité annulaire, est balayée par le sang et évite la stase à ce niveau.

Dans la document FR—A—2 515 506, on a décrit une prothèse à charnière à deux volets galbés de surface inégale dont les axes de rotation des volets sont placés à deux niveaux différents dans l'anneau. Du fait de cette disposition, le grand volet, dont l'axe est placé en amont de celui du petit volet, s'ouvre avant ce dernier, provoquant l'ouverture du petit. Ce positionnement est destiné à réduire les turbulences fatales au moment de l'ouverture d'un volet.

Quel que soit le type de prothèse à clapet (sans ou avec charnière) le blockage du volet en position ouverte parallèlement à l'axe du courant, est un accident grave connu. Pour l'éviter, il est conseillé de galber le volet ou/et d'en limiter l'ouverture. Mais le risque d'un profil trop galbé est de profoquer un effet de sténose. Le même risque est encouru en réduisant par des butées l'ouverture du volet pour éviter qu'il n'atteigne la position d'ouverture totale. Enfin, les clapets à charnière posent le problème technique de leur introduction et de leur mise en place dans un anneau rigide. Toutes les solutions proposées ont leurs inconvénients et leurs risques.

L'invention a pour but d'améliorer le fonctionnement hémodynamique et de réduire les turbulences, source de thrombose, dans les prothèses mécaniques à clapet.

Le dessin annexé, donné à titre d'exemple, permettra de mieux comprendre l'invention, les caractéristiques qu'elle présente et les avantages qu'elle est susceptible de procurer:

Fig. 1 est une coupe longitudinale centrale d'un registre basculant établi conformément à l'invention à sa position de fermeture.

Fig. 2 est une vue semblable à celle de fig. 1, mais montrant le volet ouvert.

Fig. 3 et 4 sont des coupes transversales réalisées dans le plan du pivot du volet en fig. 1 et 2.

Fig. 5 illustre une variante comportant deux volets, dont l'articulation est réalisée conformément à l'invention.

Fig. 6 en est une coupe transversale par le plan contenant les pivots des deux volets.

Fig. 7 montre une variante de réalisation.

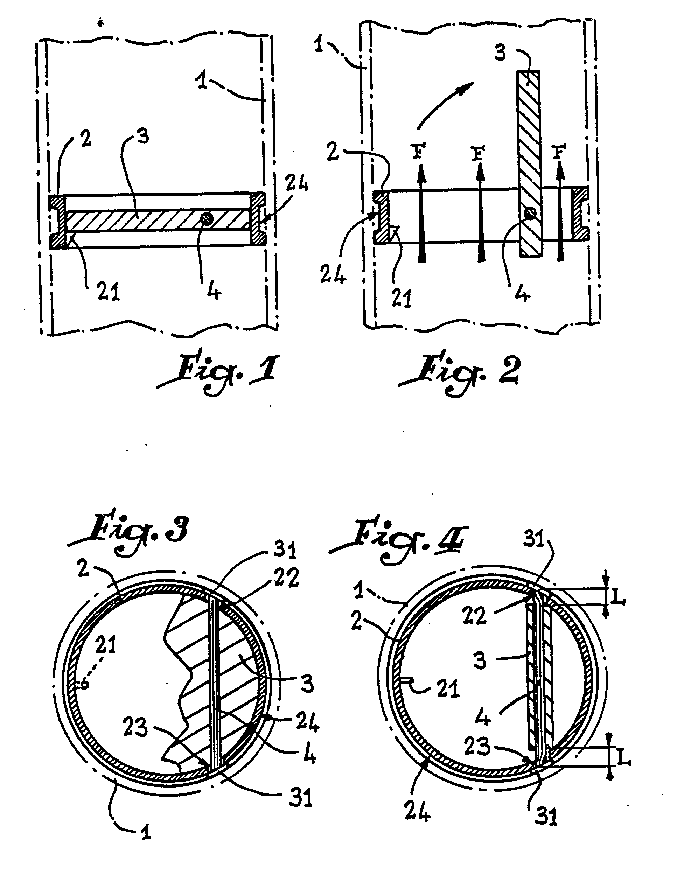

Les modes de réalisation de fig. 1 à 4 concernent plus particulièrement mais non exclusivement, une application industrielle de l'invention.

Dans une canalisation 1, illustrée en traits discontinus, on a placé un anneau 2 présentant le même profil en plat, que celui de la canalisation 1 et dans lequel est monté un disque 3 de section correspondante à celle intérieure dudit anneau et basculant autour d'un pivot 4. En position de repos, le dirsque 3 repose sur une butée 21 de l'anneau 2.

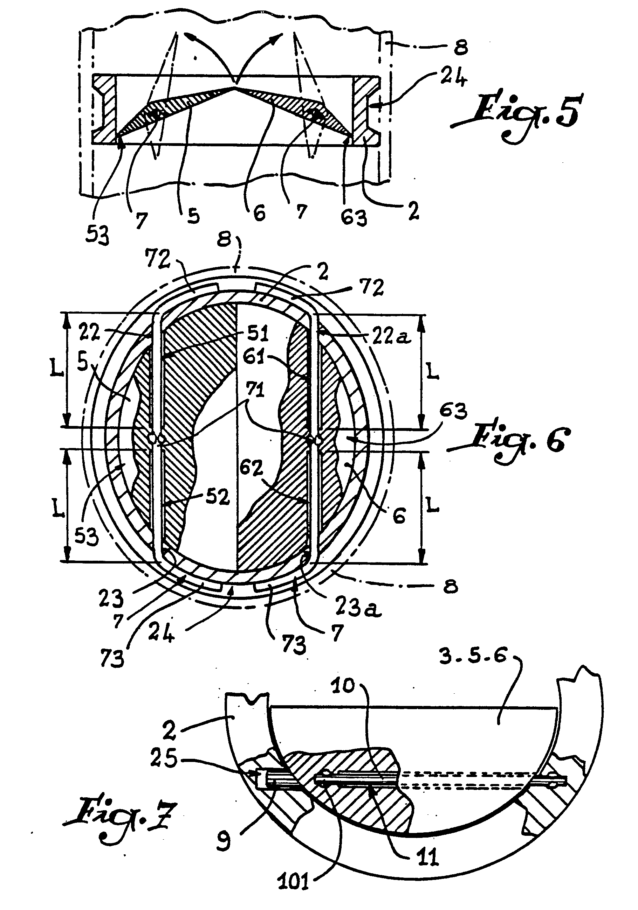

Comme montré en fig. 2, si un fluide s'écoule dans la canalisation 1 dans le sens des flèches F, le disque ou papillon 3 est déplace par la force du courant et il vient s'orienter dans la direction de celui-ci, c'est-à-dire sensiblement axialement par rapport à la canalisation en question.

Le problème qui se pose est le retour du disque ou papillon 3 à sa position initiale quand le courant de fluide cesse.

Conformèment à l'invention, le pivot 4 est réalisé sous la forme d'une tige en matière élastique telle qu'un acier à ressort, dont la partie coopérant avec le disque 3 est assujettie par soudure, poinçonnage ou autrement au disque considéré. Le pivot est excentré par rapport audit disque. Ses parties dépassant au-delà de la périphérie de celui-ci pénètrent dans deux alésages 22—23 de même axe géométrique ménangé dans l'anneau 2, puis les bouts du pivot 4 sont angulairement fixés à l'extériur de l'anneau 2. On réalise ainsi une double barre de torsion sur les parties du pivot 4 se trouvant dans l'épaisseur de l'anneau 2. Les alésages 22 et 23 forment d'une part palier pour le rotation du pivot 4 et d'autre part espace libre dans lequel celui-ci peut jouer son rôle de barre de torsion. Bien entendu, le diamètre du pivot 4 est déterminé de telle manière que le disque ou volet 3 puisse s'ouvrir sous l'effet de la pression engendrée par le passage du fluide.

Il va de soi que le pivot 4 pourrait être réalisé en deux parties angulairement assujetties d'une part au disque et d'autre part à l'extérieur de l'anneau 2, de manière qu'il existe deux barres de torsion alignées. On note que chaque partie extrême du pivot 4 peut être assujettie par tout moyen à l'extérieur de l'anneau par exemple en l'engageant dans l'alésage cannelé d'un bossage 31 solidaire de la périphérie dudit anneau, les parties extrêmes en question étant également cannelées.

Dans un mode de réalisation particulièrement intéressant de l'invention, on utilise la fonction de barre de torsion dans une prothèse valvulaire cardiaque illustrée très schématiquement en fig. 5 et 6.

Dans cette application, on utilise un, deux ou plusieurs volets. Dans l'exemple schématique des fig. 5 et 6, on emploie deux volets, référencés 5 et 6, l'anneau 2 présentant dans ce cas, mais de manière non limitative, une forme elliptique en profil en plan. Dans cette application, le pivot de chaque volet 5, 6 est réalisé sous la forme d'un fil 7, propre à développer une réaction de retour à sa position initiale quand il subit une torsion. Un tel fil doit être en outre biocompatible, biostable et non dégradable, qualités indispensables pour des applications cardio-vasculaires. Il est par exemple choisi parmi les matériaux employés pour les sutures chirurgicales non dégradables, tels que ceux existant dans le commerce, plastiques, métaux, composites, fibres de carbone, etc....

Comme dans l'application industrielle ci-dessus, chaque volet 5, 6 est assujetti en rotation par exemple par surmoulage sur une partie déformée localement au tronçon central 71 du fil 7. Dans l'application cardiaque donnée en exemple non limitatif, il existe sur l'anneau deux paires d'alésages correspondant 22—23 qui, dans le cas présent, ont été référencées respectivement 22 et 23 en vis-à-vis du volet 5 et 22a, 23a en vis-à-vis de celui 6. Là encore, la partie du fil 7 qui dépasse hors de chaque volet 5, 6 transverse librement les alésages considérés, de manière que ceux-ci constituent des paliers pour la rotation dudit fil lors du basculement des volets. Les deux bouts libres 72, 73 de chaque fil 7 sont ensuite appliqués contre la face externe de l'anneau 2 où ils sont fixés par tout moyen, par exemple par soudure à ultra-sons, dans une gorge 24. On obtient ainsi le même résultat que celui atteint par le pivot en acier à ressort 4, décrit en référence aux fig. 1 à 4.

Pour éviter le réfléchissement du fil, on peut le soumettre à une traction avant son assujettissement à l'anneau.

Si l'on désire une longueur L importante de torsion du fil, il suffit de prévoir de faire passer le fil dans des chambrages 51, 52 respectivement 61, 62 ménagés dans les volets et débouchant sur la périphérie de ceux-ci pour laisser toutefois un tronçon central 71 de longueur bien suffisante pour l'assujettissement en rotation du fil par rapport à chaque volet.

On notera que la position de fermeture des volets peut être limitée assi par butée de leur partie périphérique 53, 63 contre la paroi intérieure de l'anneau 2 (fig. 5).

Le fil élastique 7 étant de faible diamètre, les risques de turbulences et de coagulation au niveau de la charnière sont réduits. Il est également possible de réaliser des prothèses de petites dimensions pour des petits orifices. On a représenté en fig. 5 et 6 une valve à deux volets, mais elle pourrait en comprendre un seul ou plus de deux. De même la forme de l'anneau 2 qui a été représentée elliptique, pourrait être circulaire ou de tout autre profil approprié. Les prothèses valvulaires cardiaques ainsi décrites, destinées à remplacer les orifices du coeur peuvent aussi être placées dans un vaisseau artificiel 8 illustré en traits discontinus en fig. 5 et 6 afin d'être utilisé pour certains pontages entre les ventricules et les gros vaisseaux ou dans un coeur articiel par une fixation adaptée.

Là encore le fil 7 a un triple rôle:

1° — Il maintient le volet ou les volets en position adéquate dans l'orifice de l'anneau 2.

2° — Il joue le rôle de charnière.

3° — Grâce à son élasticité, il accélère la fermeture du volet ou des volets par effet de torsion.

On notera que suivant la position du ou des volets lors de l'assujettissement du fil à l'anneau, on accélère soit la femeture du volet après arrêt de l'écoulement du fluide, soit au contraire son ouverture.

Au moyen d'une torsion convenable du fil au moment du montage, on peut créer une précontrainte favorable à l'ouverture ou à la fermeture du ou des volets.

Grâce à la structure suivant l'invention, les effets du couple de torsion peuvent être facilement maîtrisés à volonté, afin d'éviter toute gêne à l'écoulement du fluide. Là encore, on peut favoriser la fermeture ou l'ouveture du ou des volets comme indiqué ci-dessus.

En admettant que par vieillissement l'élasticité du fil diminue, cette variation de condition mécanique ne présentera aucun inconvénient, puisqu'alors ce fil ne constituera plus qu'un pivot de maintien des volets du genre de ceux utilisés jusqu'à présent dans la pratique.

En position d'ouverture totale, le ou les volets placés dans l'axe du courant de fluide ne risquent pas de rester bloqués, et de provoquer un accident cardiaque irrémédiable. La force de torsion du fil élastique étant maximale en pleine ouverture, grâce à l'invention on obtient un dispositif de rappel supérieur au moyen magnétique déjà utilisé dont l'attraction diminue avec la distance et se fait sentir donc surtout au moment où les deux surfaces magnétisées sont très proches l'une de l'autre, c'est-à-dire vers la fermeture des volets.

Dans la variante de fig. 7, le volet 3 ou chaque volet 5, 6 comporte un pivot 9 engagé dans un trou 25 de l'anneau 2 et un seul élément élastique 10 de grande longueur disposé sur le même axe géométrique. L'extrémité intérieure 101 de l'élément 10 est ancrée dans le volet, le reste de cet élément traversant un chambrage 11 ménagé dans le volet de venir s'ancrer dans l'anneau comme indiqué plus haut. Une telle structure permet d'obtenir un couple de rappel très important dû à la longueur sur laquelle s'effectue la déformation angulaire.

En particulier, il va de soi que les éléments élastiques, comme celui 4, pourraient être réalisés en deux parties disposées sur le même axe géométrique et dont les deux extrémités seraient angulairement assujetties respectivement au volet correspondant et à l'extérieur de l'anneau. En outre, il est bien évideent qu'en déplaçant l'emplacement des pivots 7, on peut obtenir soit une ouverture centrale (fig. 5 et 6) soit une ouverture latérale, par exemple en les rapprochant du centre de l'anneau 2.

## Revendications

1. Valve à au moins un volet basculant articulé autour de pivots par rapport à un anneau fixe (2), caractérisée en ce que le volet (3) est angulairement fixe par rapport à au moins un élément élastique funiculaire (4) dont les extrémités dépassent vers l'extérieur de ce volet pour traverser au mons en partie l'anneau (2) afin d'y pivoter, les extrémités libres de l'élément (4) étant ancrées par rapport à l'anneau (2) de manière à constituer deux barres de torsion ramenant le volet (3) à sa position d'origine après que celui-ci ait été déplacé.

2. Valve suivant la revendication 1, caractérisée en ce que l'élément élastique est une tige formant ressort de torsion pivotant dans son passage (21—22) dans l'anneau et dont les extrémités sont angulairement fixées respectivement par rapport à l'extérieur dudit anneau (2).

3. Valve suivant la revendication 1, notamment pour la réalisation de prothèses valvulaires cardiaques, caractérisée en ce que l'élément élastique (4) est réalisé sous la forme d'un fil (7) propre à développer une réaction de retour à sa position initiale quand il subit une torsion, dont le tronçon central (71) est noyé dans chaque volet (3, 5, 6) afin de lui être assujetti angulairement, tandis qu'après avoir traversé deux alésages (22, 23, 22a, 23b) opposés ménagés dans l'anneau (2), ses deux bouts libres (72, 73) sont fixés à l'anneau (2).

4. Valve suivant la revendication 3, utilisée comme prothèse valvulaire cardiaque, caractérisée en ce qu'elle comprend deux volets (5, 6) qui comportent chacun un fil (7) dont les extrémités des bouts libres (72, 73) dépassant hors de l'anneau (2) sont associés à la paroi extérieure de celui-ci pour les immobiliser angulairement.

5. Valve suivant la revendication 3, utilisée comme prothèse valvulaire cardiaque, caractérisée en ce que la longueur du tronçon central (71) du fil (7) noyé dans chaque volet (3, 5, 6) est variable.

6. Valve suivant la revendication 3, utilisée comme prothèse valvulaire cardiaque, caractérisée en ce que des butées sont prévues pour les volets (5, 6) à leur position de fermeture.

7. Valve suivant la revendication 3, utilisée comme prothèse valvulaire cardiaque, caractérisée en ce que les volets (5, 6) sont articulés dans une zone diamétrale de l'anneau (2) afin que leur ouverture soit latérale.

8. Valve suivant la revendication 3, utilisée comme prothèse valvulaire cardiaque, caractérisée en ce que les volets (5, 6) sont articulés à l'anneau (2) afin que leur ouverture détermine une zone libre centrale dans cet anneau.

9. Dispositif suivant l'une quelconque des revendications précédentes, caractérisée en ce que l'élément funiculaire élastique (4, 7) est réalisé au moyen de deux organes alignés dont les extrémités libres sont angulairement assujetties au volet (3, 5, 6) et à l'extérieur de l'anneau (2).

10. Dispositif suivant la revendicaiton 9, caractérisée en ce que l'un des éléments funiculaires est un pivot rigide.

11. Dispositif suivant l'une quelconque des revendications précédentes, caractérisée en ce qu'au moins l'un des éléments funiculaires élastiques subit lors du montage une précontrainte de torsion.

## Patentansprüche

1. Ventil mit mindestens einer Schwenkklappe, die in bezug auf einen fest angeordneten Ring (2) um Schwenkachsen schwenkbar gelagert ist, dadurch gekennzeichnet, daß die Klappe (3) winkelmäßig in bezug auf mindestens ein elastisches Draht- bzw. Seilelement (4) feststehend ist, dessen Enden über die Klappe hinausragen und zumindest teilweise den Ring (2) durchqueren, um darin zu schwenken, wobei die freien Enden des Elementes (4) in bezug auf den Ring (2) derart verankert sind, daß sie zwei Torsionsstäbe bzw. -federn bilden, die die Klappe (3) in ihre Ursprungsstellung zurückbringen, nachdem sie verschwenkt wurde.

2. Ventil nach Anspruch 1, dadurch gekennzeichnet, daß das elastische Element eine Stange ist, die eine in dem Durchgang (21—22) im Ring schwenkende Torsionsfeder bildet, deren Enden jeweils in bezug auf das Äußere des Ringes (2) winkelmäßig fest angeordnet sind.

3. Ventil nach Anspruch 1 insbesondere zur Realisierung von Herzklappenprothesen, dadurch gekennzeichnet, daß das elastische Element (4) als Faden (7) ausgebildet ist, der geeignet ist, eine Umkehrreaktion in seine Ursprungsstellung zu entwickeln, wenn er einer Torsion unterworfen wird, wobei sein Mittelstück (71) in eder Klappe (3, 5, 6) eingebettet ist, um mit ihr winkelmäßig festverbunden zu sein, während seine freien Enden nach Durchquerung zweier gegenüberliegenden im Ring (2) vorgesehenen Bohrungen (22, 23, 22a, 23b) an dem Ring (2) befestigt sind.

4. Ventil nach Anspruch 3 bei Verwendung als Herzklappenprothese, dadurch gekennzeichnet, daß zwei Klappen (5, 6) vorgesehen sind, die jeweils einen Faden (7) aufweisen, deren über den Ring (2) hinausgehenden freien Enden (72, 73) mit dessen äußeren Wand verbunden sind, um sie winkelmäßig festzulegen.

5. Ventil nach Anspruch 3 bei Verwendung als Herzklappenprothese, dadurch gekennzeichnet, daß die Länge des Mittelstücks (71) des in jeder Klappe (3, 5, 6) eingebetteten Fadens (7) variabel ist.

6. Ventil nach Anspruch 3 bei Verwendung als Herzklappenprothese, dadurch gekennzeichnet, daß Anschläge für die Klappen (5, 6) in ihrer Schließstellung vorgesehen sind.

7. Ventil nach Anspruch 3 bei Verwendung als Herzklappenprothese, dadurch gekennzeichnet, daß die Klappen (5, 6) in einem diametralen Bereich des Ringes (2) gelagert sind, damit ihre Öffnung seitlich erfolgt.

8. Ventil nach Anspruch 3 bei Verwendung als Herzklappenprothese, dadurch gekennzeichnet, daß die Klappen (5, 6) an dem Ring (2) angelenkt sind, damit ihre Öffnung einen freien mittleren Bereich in diesem Ring bestimmt.

9. Vorrichtung nach einem der vorhergehenden Ansprüche, dadurch gekennzeichnet, daß das elastische Draht- bzw. Seilelement (4, 7) mittels zweier ausgerichteter Bauteile realisiert ist, deren freien Enden winkelmäßig fest mit der Klappe (3, 5, 6) und mit dem Äußeren des Ringes (2) verbuden sind.

10. Vorrichtung nach Anspruch 9, dadurch gekennzeichnet, daß eines der Draht- bzw. Seilelemente eine starre Schwenkachse ist.

11. Vorrichtung nach einem der vorhergehenden Ansprüche, dadurch gekennzeichnet, daß mindestens eines der elastischen Seilemente während der Montage einer Torsionsvorrspannung unterworfen wird.

## Claims

1. Valve with at least one tilting flap articulated about pivots with respect to a fixed ring (2), characterised in that the flap (3) is at a fixed angle with respect to at least one funicular resilient element (4) whereof the ends project towards the outside of this flap to cross at least partially the ring (2) in order to pivot thereon, the free ends of the element (4) being anchored with respect to the ring (2) so as to form two torsion bars returning the flap (3) to its original position after the latter has been displaced.

2. Valve according to Claim 1, characterised in that the resilient element is a rod forming a torsional spring pivoting within its passage (21—22) in the ring and whose ends are respectively at a fixed angle with respect to the outside of the said ring (2).

3. Valve according to Claim 1, in particular for producing cardiac valve prostheses, characterised in that the resilient element (4) is produced in the form of a filament (7) suitable for producing a reaction of return motion to its initial position when it undergoes torsion, whereof the central section (71) is embedded in each flap (3, 5, 6) in order to be fixed thereto at an angle, whereas, after having crossed two opposing bores (22, 23, 22a, 23b) made in the ring (2), the two free ends (72, 73) of the filament are fixed to the ring (2).

4. Valve according to Claim 3, used as a cardiac valve prosthesis, characterised in that it comprises two flaps (5, 6) each having a filament (7) whereof the extremities of the free ends (72, 73) projecting outside the ring (2) are associated with the outer wall thereof to keep them stationary at an angle.

5. Valve according to Claim 3, used as a cardiac valve prosthesis, characterised in that the length of the central section (71) of the filament (7) embedded in each flap (3, 5, 6) is variable.

6. Valve according to Claim 3, used as a cardiac valve prosthesis, characterised in that stops are provided for the flaps (5, 6) in their closed position.

7. Valve according to Claim 3, used as a cardiac valve prosthesis, characterised in that the flaps (5, 6) are articulated in a diametral region of the ring (2) in order that they open laterally.

8. Valve according to Claim 3, used as a cardiac valve prosthesis, characterised in that the flaps (5, 6) are articulated to the ring (2) in order that opening them defines a central free region in this ring.

9. Device according to any one of the preceding claims characterised in that the resilient funicular element (4, 7) is produced by means of two aligned members whereof the free ends are fixed to the flap (3, 5, 6) at an angle and outside the ring (2).

10. Device according to Claim 9, characterised in that one of the funicular elements is a rigid pivot.

11. Device according to any one of the preceding claims characterised in that at least one of the resilient funicular elements undergoes a torsional pre-stressing during assembly.

Fig. 1

Fig. 2

Fig. 3

Fig. 4

*Fig.5*

*Fig. 6*

*Fig.7*